# FASCICULE DE BREVET EUROPEEN

(11) **EP 1 213 008 B1**
(45) Date de publication et mention de la délivrance du brevet: **15.12.2004**
(21) Numéro de dépôt: 01402697.5
(22) Date de dépôt: 18.10.2001
(51) Int. Cl.: A61K 7/00, A61K 7/48

(54) **Composition de soin ou de maquillage contenant des fibres et un copolymère dispersant**
Zusammensetzung für die Körperpflege oder das Schminken, die Fasern und ein Copolymer Dispergiermittel enthält
Make-up or bodycare composition containing fibres and a dispersing copolymer

(30) Priorité: 05.12.2000 FR 0015741
(43) Date de publication de la demande: 12.06.2002
(73) Titulaire: L'OREAL, 75008 Paris (FR)
(72) Inventeur: Afriat, Isabelle, 75003 Paris (FR); Tournilhac, Florence, 75011 Paris (FR)
(74) Mandataire: Rasson, Catherine

(56) Documents cités:
- WO-A-00/06114
- US-A- 5 965 146

## Description

La présente invention se rapporte à une composition pour application topique contenant des fibres et un copolymère comportant des groupements carboxylates et des groupements polydiméthylsiloxanes, et à ses utilisations notamment dans les domaines cosmétique et dermatologique, en particulier pour le soin, le traitement ou le maquillage des matières kératiniques telles que la peau, y compris le cuir chevelu, les lèvres du visage et les phanères comme les cils, les sourcils, les ongles et les cheveux. La présente invention se rapporte aussi à l'utilisation d'un copolymère comportant des groupements carboxylates et des groupements polydiméthylsiloxanes, dans une composition contenant des fibres, comme agent dispersant et notamment pour assurer une dispersion homogène des fibres dans la dite composition, pour stabiliser ladite composition, et pour obtenir un dépôt homogène de la dite composition sur les matières kératiniques.

Il est connu d'incorporer des fibres dans les compositions cosmétiques. Ainsi, le document JP07-196440 décrit des compositions cosmétiques contenant des fibres de polyamide courtes, celles-ci donnant aux dites compositions un toucher velouté et une bonne tenue cosmétique. Par ailleurs, dans le domaine du maquillage de la peau, il est connu d'utiliser des fibres dans des produits de maquillage notamment pour leurs effets allongeant dans des mascaras (voir JP-A-57/158714), leur toucher « textile» (voir JP-A-7/196440), leur effet de tissus ou encore leurs propriétés hydratantes dans des rouges à lèvres (voir le document US-A-5 498 407) ou pour améliorer les contours du rouge à lèvres sur les bords des lèvres (voir le document EP-A-0 106 762).

Malheureusement, il est difficile de disperser des fibres dans des compositions pour application topique, de façon homogène et sans former d'amas, notamment lorsque ces compositions sont sous forme d'émulsions, et cette hétérogénéité de la dispersion confère généralement une hétérogénéité lors de l'application de la composition sur la peau, et, lorsqu'il s'agit d'une composition colorée et en particulier de maquillage, un maquillage non uniforme et peu esthétique. En outre, cette difficulté de dispersion conduit à des compositions instables et donc difficilement commercialisables.

Il subsiste donc le besoin d'une composition pour application topique, et notamment sous forme d'émulsion, contenant des fibres et ne présentant pas les inconvénients ci-dessus, c'est-à-dire présentant une bonne stabilité et conférant un maquillage ou un soin homogène et esthétique.

L'invention a justement pour objet une composition destinée notamment au soin et/ou au traitement et/ou au maquillage des matières kératiniques permettant de remédier à ces inconvénients. De façon surprenante, le demandeur a trouvé que l'utilisation d'un copolymère comportant des groupements carboxylates et des groupements polydiméthylsiloxanes, permettait l'obtention d'une composition ayant une très bonne stabilité et de bonnes propriétés cosmétiques.

L'invention s'applique non seulement aux produits de soin, de traitement et/ou de maquillage de la peau y compris du cuir chevelu, aussi bien du visage que du corps humain, et des lèvres du visage, mais aussi aux produits de maquillage des phanères comme les cils, les sourcils et les ongles, et aux produits de soin et/ou de traitement des cheveux.

De façon plus précise, l'invention a pour objet une composition pour application topique comprenant dans un milieu physiologiquement acceptable, des fibres et au moins un copolymère comportant des groupements carboxylates et des groupements polydiméthylsiloxanes.

On entend ici par « application topique » une application externe sur les matières kératiniques, que sont notamment la peau, le cuir chevelu, les cils, les sourcils, les ongles, les muqueuses et les cheveux.

La composition obtenue présente une agréable douceur lors de son application sur les matières kératiniques et notamment sur la peau, et elle présente une bonne tenue cosmétique. En outre, le copolymère utilisé dans la composition de l'invention permet une dispersion homogène des fibres et assure la stabilité de la composition dans le temps. Par ailleurs, il permet d'obtenir un dépôt plus homogène de la dite composition sur les matières kératiniques et notamment sur la peau, le cuir chevelu, les cils, les sourcils, les ongles, les muqueuses et les cheveux.

Aussi, l'invention a encore pour objet l'utilisation d'un copolymère comportant des groupements carboxylates et des groupements polydiméthylsiloxanes, dans une composition contenant des fibres, pour assurer une dispersion homogène des fibres dans la dite composition et pour stabiliser ladite composition.

L'invention a encore pour objet l'utilisation d'un copolymère comportant des groupements carboxylates et des groupements polydiméthylsiloxanes, dans une composition contenant des fibres, comme agent dispersant.

Enfin, l'invention a encore pour objet l'utilisation cosmétique d'un copolymère comportant des groupements carboxylates et des groupements polydiméthylsiloxanes, dans une composition cosmétique contenant des fibres, pour obtenir un dépôt homogène de la dite composition sur les matières kératiniques.

Par "fibre", il faut comprendre dans la présente demande, un objet de longueur L et de diamètre D, tel que L soit très supérieur à D, D étant le diamètre du cercle dans lequel s'inscrit la section de la fibre. En particulier, le rapport L/D (ou facteur de forme) est choisi dans la gamme allant de 3,5 à 2 500, de préférence de 5 à 500 et mieux de 5 à 150.

Les fibres utilisables dans la composition de l'invention peuvent être des fibres hydrophiles ou hydrophobes, d'origine synthétique ou naturelle, minérale ou organique.

Ces fibres peuvent être courtes ou longues, unitaires ou organisées par exemple tressées. Leur forme ou morphologie peut être quelconque et notamment de section circulaire ou polygonale (carrée, hexagonale ou octogonale) selon l'application spécifique envisagée. En particulier, leurs extrémités sont épointées et/ou polies pour éviter de se blesser. Elles peuvent être multilobées et notamment trilobées.

En particulier, les fibres peuvent avoir une longueur (L) allant de 1 µm (0,001 mm) à 10 mm et de préférence de 0,1 µm à 5 mm. Leur section peut être comprise dans un cercle de diamètre (D) allant de 1 nm (0,001 µm) à 100 µm, de préférence allant de 1 nm (0,001 µm) à 50 µm et mieux de 5 µm à 40 µm.

Le titre des fibres est souvent donné en denier ou décitex. Le denier est le poids en gramme pour 9 km de fil. De préférence, les fibres utilisées selon l'invention ont un titre allant de 0,15 à 30 deniers, et mieux de 0,18 à 18 deniers.

Le facteur de forme, le titre et la morphologie des fibres sont les trois facteurs importants pour définir une fibre.

Les fibres peuvent être celles utilisées dans la fabrication des textiles et notamment des fibres de soie, de coton, de liège, de canne à sucre, de laine, de lin, de cellulose extraites notamment du bois, des légumes ou des algues, de polyamide (Nylon®), de cellulose modifiée (rayonne, viscose, acétate notamment d'acétate de rayonne), de poly-(p-phénylène téréphtalamide) notamment de Kevlar®, en acrylique notamment de polyméthacrylate de méthyle ou de polyméthacrylate de 2-hydroxyéthyle, de polyoléfine et notamment de polyéthylène ou de polypropylène, de verre, de silice, d'aramide, de carbone notamment sous forme graphite, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-dessus, comme des fibres de polyamide/polyester.

On peut aussi utiliser les fibres synthétiques résorbables utilisées en chirurgie, comme les fibres préparées à partir d'acide glycolique et de caprolactone (Monocryl de la société Johnson & Johnson) ; les fibres synthétiques résorbables du type copolymère d'acide lactique et d'acide glycolique (Vicryl de la société Johnson & Johnson) ; les fibres de polyester téréphtalique (Ethibond de la société Johnson & Johnson) et les fils d'acier inoxydable (Acier de la société Johnson & Johnson).

Par ailleurs, les fibres peuvent être traitées ou non en surface, enrobées ou non, ou à la fois traitées et enrobées. Comme fibres enrobées et/ou traitées utilisables dans l'invention, on peut citer des fibres de polyamide enrobées de sulfure de cuivre pour un effet antistatique (par exemple R-STAT de la société Rhodia) ou un autre polymère permettant une organisation particulière des fibres (traitement de surface spécifique) ou traitement de surface induisant des effets de couleurs/hologrammes (fibre Lurex de la société Sildorex, par exemple).

On peut aussi utiliser des mélanges des fibres citées ci-dessus.

Selon leurs propriétés, les fibres utilisées selon la présente invention peuvent être introduites dans un milieu aqueux, un milieu huileux ou dans une poudre.

Les fibres utilisables selon l'invention sont de préférence choisies parmi les fibres de polyamide, les fibres de poly-(p-phénylènetéréphtalamide), les fibres de coton et leurs mélanges. Leur longueur peut aller de 0,1 à 10 mm, de préférence de 0,1 à 5 mm, leur diamètre moyen peut aller de 5 à 50 µm et le facteur de forme va de préférence de 5 à 150.

En particulier, on peut utiliser les fibres de polyamide commercialisées par les Etablissements P. Bonte sous le nom Polyamide 0,9 Dtex, ayant un diamètre moyen de 15 à 20 µm, un titre d'environ 0,9 dtex (0,81 denier) et une longueur allant de 0,3 mm à 5 mm (Polyamide 0,9 Dtex 0,3 mm, Polyamide 0,9 Dtex 3 mm ou Polyamide 0,9 Dtex 5 mm). On peut aussi utiliser les fibres de poly-(p-phénylène téréphtalamide) de diamètre moyen de 12 µm et de longueur d'environ 1,5 mm comme celles vendues sous le nom de Kevlar Floc par la société Du Pont Fibres. Ces fibres de polyamide sont de préférence introduites dans un milieu huileux.

On peut aussi utiliser des fibres de coton ayant un diamètre moyen de 20 µm, une longueur allant de 0,3 mm, et un facteur de forme de 15.

La quantité de fibres dans la composition selon l'invention peut varier dans une large mesure selon l'application spécifique et le type de produit envisagé. Elle peut aller par exemple de 0,1 à 50 % en poids, de préférence de 0,5 à 30 % en poids et mieux de 2 à 15 % en poids par rapport au poids total de la composition. Pour un produit de maquillage du visage du type fond de teint ou des lèvres (du type rouge à lèvres) la concentration en fibres peut aller de 0,1 à 20 % du poids total de la composition, de préférence de 0,5 à 10 % du poids total de la composition. Pour un effet spécial notamment de maquillage du corps, des ongles ou des cheveux ou pour un produit de soin du visage, la quantité de fibres peut aller jusqu'à 30 % du poids total de la composition.

Par « copolymère comportant des groupements carboxylates et des groupements polydiméthylsiloxanes », on entend dans la présente demande, un copolymère obtenu à partir de (a) un ou plusieurs monomères carboxyliques (acide ou ester), et (b) une ou plusieurs chaînes polydiméthylsiloxane (PDMS).

On entend dans la présente demande par « monomère carboxylique » aussi bien les monomères d'acide carboxylique que les monomères d'ester d'acide carboxylique. Ainsi, le monomère (a) peut être choisi par exemple parmi l'acide acrylique, l'acide méthacrylique, l'acide maléique, l'acide fumarique, l'acide itaconique, l'acide crotonique, leurs esters et les mélanges de ces monomères. Comme esters, on peut citer les monomères suivants : acrylate, méthacrylate, maléate, fumarate, itaconoate et/ou crotonoate. Selon un mode préféré de réalisation de l'invention, les monomères sous forme d'esters sont plus particulièrement choisis parmi les acrylates et méthacrylates d'alkyle linéaire ou ramifié de préférence en C₁-C₂₄ et mieux en C₁-C₂₂, le radical alkyle étant préférentiellement choisi parmi les radicaux méthyle, éthyle, stéaryle, butyle, éthyl-2-hexyle, et leurs mélanges.

Ainsi, selon un mode particulier de réalisation de l'invention, le copolymère comprend comme groupements carboxylates, au moins un groupement choisi parmi l'acide acrylique, l'acide méthacrylique, les acrylates ou méthacrylates de méthyle, d'éthyle, de stéaryle, de butyle, d'éthyl-2-hexyle, et leurs mélanges.

Dans la présente demande, on entend désigner par « polydiméthylsiloxanes » (appelé aussi organopolysiloxanes ou, en abréviation, PDMS), en conformité avec l'acceptation générale, tout polymère ou oligomère organosilicié à structure linéaire, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), comportant des radicaux triméthyle directement liés par l'intermédiaire d'un atome de carbone sur lesdits atomes de silicium. Les chaînes PDMS pouvant être utilisées pour obtenir le copolymère utilisé selon l'invention comportent au moins un groupe radical polymérisable, de préférence situé sur au moins l'une des extrémités de la chaîne, c'est-à-dire que le PDMS peut avoir par exemple un groupe radical polymérisable sur les deux extrémités de la chaîne ou avoir un groupe radical polymérisable sur une extrémité de la chaîne et un groupement terminal triméthylsilyle sur l'autre extrémité de la chaîne. Le groupe radical polymérisable peut être notamment un groupe acrylique ou méthacrylique, en particulier un groupe CH₂ = CR₁ - CO - O - R₂, où R₁ représente un hydrogène ou un groupe méthyle, et R₂ représente -CH₂-, - (CH₂)ₙ- avec n = 3, 5, 8 ou 10, -CH₂-CH(CH₃)-CH₂- , -CH₂-CH₂-O-CH₂-CH₂-, -CH₂-CH₂-O-CH₂-CH₂-CH(CH₃)-CH₂-, -CH₂-CH₂-O-CH₂ CH₂-O-CH₂-CH₂-CH₂-.

Les copolymères utilisés dans la composition de l'invention sont généralement obtenus selon les méthodes usuelles de polymérisation et de greffage, par exemple par polymérisation radicalaire (A) d'un PDMS comportant au moins un groupe radical polymérisable (par exemple sur l'une des extrémités de la chaîne ou sur les deux) et (B) d'au moins un monomère carboxylique, comme décrit par exemple dans les documents US-A-5,061,481 et US-A-5,219,560.

Les copolymères obtenus ont généralement un poids molécule allant d'environ 3000 à 200 000 et de préférence d'environ 5000 à 100 000.

Le copolymère utilisé dans la composition de l'invention peut se présenter tel quel ou sous forme dispersée dans un solvant tel que les alcools inférieurs comportant de 2 à 8 atomes de carbone, comme l'alcool isopropylique, ou les huiles comme les huiles de silicone volatiles (par exemple cyclopentasiloxane).

Comme copolymères utilisables dans la composition de l'invention, on peut citer par exemple les copolymères d'acide acrylique et d'acrylate de stéaryle à greffons polydiméthylsiloxane, les copolymères de méthacrylate de stéaryle à greffons polydiméthylsiloxane, les copolymères d'acide acrylique et de méthacrylate de stéaryle à greffons polydiméthylsiloxane, les copolymères de méthacrylate de méthyle, méthacrylate de butyle, d'acrylate d'éthyl-2-hexyle et de méthacrylate de stéaryle à greffons polydiméthylsiloxane. On peut citer en particulier comme copolymère utilisable dans la composition de l'invention, les copolymères commercialisés par la société SHIN-ETSU sous les dénominations KP-561 (nom CTFA : acrylates/dimethicone), KP-541 où le copolymère est dispersé à 60 % en poids dans de l'alcool isopropylique (nom CTFA: acrylates/dimethicone and Isopropyl alcohol), KP-545 où le copolymère est dispersé à 30 % dans du cyclopentasiloxane (nom CTFA: acrylates/dimethicone and Cyclopentasiloxane). Selon un mode préféré de réalisation de l'invention, on utilise de préférence le KP561 ; ce copolymère n'est pas dispersé dans un solvant, mais se présente sous forme cireuse, son point de fusion étant d'environ 30°C.

La composition selon l'invention peut contenir un ou plusieurs de ces copolymères. La quantité de copolymère(s) peut aller par exemple de 0,01 à 20 % en poids de matière active, de préférence de 0,1 à 10 % en poids de matière active et mieux de 0,2 à 5 % en poids de matière active par rapport au poids total de la composition.

Le milieu physiologiquement acceptable de la composition de l'invention comprend de préférence au moins une phase huileuse. Lorsque la composition est sous forme d'émulsion, cette phase huileuse constitue la phase huileuse de l'émulsion.

La phase huileuse contient au moins un corps gras choisi parmi les huiles liquides à température ambiante (20-25°C), volatiles ou non, les cires, les gommes et les corps gras pâteux, d'origine animale, végétale, minérale ou synthétique, et leurs mélanges. La phase huileuse contient de préférence au moins une huile.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- des alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclopentasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthylsiloxysilicates, et les polyméthylphénylsiloxanes ;
- leurs mélanges.

Les autres corps gras pouvant être présents dans la phase huileuse sont par exemple les acides gras comportant de 8 à 30 atomes de carbone, comme l'acide stéarique, l'acide laurique, l'acide palmitique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la Trifluoropropyldimethicone, et les élastomères de silicone comme les produits commercialisés sous les dénominations "KSG" par la société Shin-Etsu, sous la dénomination "Trefil" par la société Dow Corning ou sous les dénominations "Gransil" par la société Grant Industries.

Ces corps gras peuvent être choisis de manière variée par l'homme du métier afin de préparer une composition ayant les propriétés, par exemple de consistance ou de texture, souhaitées.

La quantité de phase huileuse dans la composition de l'invention peut aller de 0,5 à 99,89 % du poids total de la composition, de préférence de 2 à 80 % et mieux de 5 à 70 %.

La phase huileuse de la composition de l'invention peut contenir éventuellement un ou plusieurs gélifiants (ou épaississants) lipophiles. Comme gélifiants lipophiles, on peut citer par exemple les argiles modifiées telles que le silicate de magnésium modifié (bentone gel VS38 de RHEOX), l'hectorite modifiée par le chlorure de distéaryl diméthyl ammonium (nom CTFA : Disteardimonium hectorite) commercialisé sous la dénomination « bentone 38 CE » par la société RHEOX. Les agents épaississants lipophiles peuvent être présentes à raison de 0 à 30 % du poids total de la composition, de préférence 0,5 à 20 %.

En outre, la composition de l'invention peut éventuellement contenir une phase aqueuse. Avantageusement, la phase aqueuse contient de l'eau et éventuellement un ou plusieurs composés miscibles au moins en partie à l'eau comme les polyols, les mono-alcools inférieurs en C₂ à C₈, tels que l'éthanol, et les cétones en C₃ à C₄ liquides à température ambiante. Par "température ambiante", il faut comprendre une température d'environ 25°C, à pression atmosphérique normale (760 mm de Hg).

Par "polyol", il faut comprendre toute molécule organique comportant au moins deux groupements hydroxyle libres. Comme polyols, on peut citer par exemple la glycérine, les glycols comme le butylène glycol, le propylène glycol, l'isoprène glycol, et les polyéthylène glycols, le sorbitol, les sucres comme le glucose.

La phase aqueuse peut contenir éventuellement un ou plusieurs gélifiants hydrophiles. Comme gélifiants hydrophiles, on peut citer par exemple les gommes polysaccharides et leurs dérivés (gomme de xanthane, carboxyméthyl-hydroxypropyl guar) ; les protéines ; les polymères acryliques et vinyliques, les polymères naturels modifiés, et leurs mélanges.

La phase aqueuse peut représenter de 0 à 98 % du poids total de la composition et de préférence de 10 à 95 % et mieux de 30 à 95 %. Quand on veut réaliser un stick, la composition ne comporte pas ou pratiquement pas de phase aqueuse (0 % ou moins de 5 % en poids).

Le ou les composés miscibles à l'eau, tels que polyols et alcools inférieurs, peuvent être présents en une quantité allant de 0 à 30 % du poids total de la composition notamment de 0,1 à 30 % et mieux en une quantité allant de 1 à 20 %.

La composition de l'invention peut comprendre, en outre, tout additif complémentaire usuellement utilisé dans le domaine concerné, tel que des matières colorantes comme les pigments, les nacres, les colorants hydrosolubles ou liposolubles, des antioxydants, des huiles essentielles, des conservateurs, des actifs cosmétiques ou dermatologiques comme des émollients, des hydratants (glycérine), des vitamines, des acides gras essentiels, des filtres solaires lipophiles ou hydrophiles, des neutralisants, des charges, des ajusteurs de pH (base ou acide), des électrolytes (comme le sulfate de magnésium ou le chlorure de sodium), des parfums et leurs mélanges. Ces additifs peuvent être présents dans la composition selon les quantités habituellement utilisées et par exemple à raison de 0 à 20% du poids total de la composition et mieux de 0,1 à 10%. Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée. En particulier, ces additifs ne devront pas nuire à l'homogénéité, à la stabilité et au confort de la composition.

Selon un mode particulier de réalisation de l'invention, la composition contient au moins une charge et/ou un agent épaississant lipophile (gélifiant).

Comme charges, on peut notamment citer le talc, le mica, la silice, le nitrure de bore, et les charges sphériques. Comme charges sphériques, on peut citer par exemple les poudres de Nylon® telles que Nylon-12 (Orgasol® commercialisé par la société Atochem), les poudres de polyéthylène, le Téflon®, l'amidon éventuellement modifié, des microsphères de copolymères, telles que celles commercialisées sous les dénominations Expancel® par la société Nobel Industrie, le Polytrap® commercialisé par la société Dow Corning, les microbilles de résine de silicone telles que celles commercialisées par la société Toshiba sous la dénomination Tospearl®, et leurs mélanges. Les charges peuvent être présentes à raison de 0 à 35 % et de préférence 0,5 à 15 % du poids total de la composition.

Selon un mode préféré de réalisation de l'invention, la composition contient au moins une charge, de préférence au moins une charge sphérique telle que notamment le Nylon-12, car l'addition de charges et notamment de charges sphériques améliore le délitage de la composition sur la peau et facilite donc le dépôt du produit sur la peau.

La composition de l'invention peut se présenter sous forme de pâte, de solide, de crème plus ou moins fluide voire même de lotion. Elle peut être sous forme d'un gel huileux ou aqueux, d'une dispersion ou émulsion huile-dans-eau ou eau-dans-huile ou multiple (E/H/E ou H/E/H), fluide, rigide ou souple, éventuellement coulé en stick ou en coupelle.

Selon un mode préféré de réalisation de l'invention, la composition se présente sous forme d'une émulsion, et plus particulièrement sous forme d'une émulsion E/H (phase aqueuse dispersée dans phase huileuse) ou H/E (phase huileuse dispersée dans phase aqueuse).

Quand la composition est une émulsion, la phase huileuse contient une ou plusieurs huiles et éventuellement d'autres corps gras, comme décrit ci-dessus. La proportion de la phase huileuse de l'émulsion peut aller par exemple de 5 à 80 % en poids, et de préférence de 5 à 50 % en poids par rapport au poids total de la composition. Les émulsionnants et éventuellement les co-émulsionnants utilisés dans la composition sous forme d'émulsion sont choisis parmi ceux classiquement utilisés dans le domaine cosmétique ou dermatologique. L'émulsionnant et éventuellement le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90^{R} par la société Goldschmidt.

Pour les émulsions H/E, on peut citer par exemple comme émulsionnants les esters d'acides gras et de glycérol oxyalkylénés (plus particulièrement polyoxyéthylénés), les esters d'acides gras et de sorbitan oxyalkylénés (plus particulièrement oxyéthylénés), les esters d'acides gras oxyalkylénés (oxyéthylénés et/ou oxypropylénés), les éthers d'alcools gras oxyalkylénés (oxyéthylénés et/ou oxypropylénés), les esters de sucres comme le stéarate de sucrose, et leurs mélanges.

La composition de l'invention peut constituer une composition dermatologique ou cosmétique, notamment destinée au soin, au traitement, au nettoyage et au maquillage des matières kératiniques et notamment de la peau, des lèvres, des cheveux, des cils et des ongles des êtres humains.

Ainsi, elle peut constituer une composition de traitement ou de soin de la peau (y compris le cuir chevelu), des fibres kératiniques (cheveux, cils, sourcils), des ongles ou des lèvres, ou une composition de protection solaire ou de bronzage artificiel, ou encore un produit nettoyant ou démaquillant de la peau, des cheveux, des sourcils ou des cils, un produit déodorant ou encore un produit parfumant. Elle est alors généralement non colorée ou faiblement colorée, et elle peut contenir éventuellement des actifs cosmétiques ou dermatologiques. Elle peut alors être utilisée comme base de soin pour la peau ou les lèvres (baumes à lèvres, protégeant les lèvres du froid et/ou du soleil et/ou du vent), comme crème de soin de jour ou de nuit pour la peau du visage et/ou du corps. Elle peut, en outre, se présenter sous forme de shampooing traitant ou non, colorant ou non, et de produit après shampooing.

La composition selon l'invention peut également constituer une composition cosmétique colorée et notamment un produit de maquillage de la peau, en particulier un fond de teint, un blush, un fard à joues ou à paupières, un mascara, un eye-liner, un produit anti-cernes en stick, un vernis à ongle, un rouge à lèvres ou un brillant à lèvres, présentant éventuellement des propriétés de soin ou de traitement, un tatouage corps.

Ainsi, l'invention a encore pour objet l'utilisation cosmétique d'une composition cosmétique telle que définie ci-dessus, pour le soin, le traitement, le nettoyage et/ou le maquillage des matières kératiniques.

Elle a aussi pour objet un procédé cosmétique de soin ou de traitement des matières kératiniques et notamment de la peau, des cheveux ou des lèvres des êtres humains, comprenant l'application sur ces matières kératiniques d'une composition en particulier cosmétique telle que définie ci-dessus.

La composition selon l'invention est préparée selon les méthodes usuelles. dans les domaines d'application considérés.

L'invention est illustrée plus en détail dans l'exemple suivant. Les composés sont indiqués selon les cas en nom CTFA ou en nom chimique, et les pourcentages sont donnés en poids sauf mention contraire.

### Exemple : Crème blanche (Emulsion E/H)

### Phase A

- Trifluoropropyl dimethicone 4 %
- Dimethicone/Dimethiconol 2,5 %
- Nylon-12 (Orgasol) 1,5 %
- Disteardimonium hectorite 3 %
- Fibres de polyamide (Polyamide 0.9 dtex, 0,3 mm - Société Paul Bonte) 12 %
- Cyclopentasiloxane 7 %

### Phase B

- Cyclopentasiloxane / Dimethicone copolyol (DC-3225 C) 10 %
- Acrylates/Dimethicone Copolymer (KP-561) 0,6 %
- Parfum 0,1 %

### Phase C

- Chlorure de sodium 5 %
- Glycérine 0,3 %
- Ethanol 2,5 %
- Conservateurs 1 %
- Eau sp 100 %

Mode opératoire : On mélange sous agitation les constituants de la phase A sans le Nylon-12 et les fibres. Par ailleurs, on mélange à chaud (environ 45 à 50°C) les constituants de la phase B sans le parfum et on agite pendant 45 minutes, puis on refroidit la phase B à la température ambiante (environ 20 à 25°C) et on y ajoute le parfum. On verse cette phase B dans la phase A sous agitation, on y ajoute le Nylon-12 et les fibres, et on homogénéise le mélange. On prépare la phase C par mélange des différents constituants sous agitation et on l'incorpore peu à peu sous agitation dans le mélange obtenu précédemment.

On obtient une crème blanche, dans laquelle les fibres sont réparties de façon homogène et qui est douce à l'application. Cette crème peut être utilisée par exemple pour le soin de la peau. Le dépôt obtenu est bien homogène.

## Revendications

1. Composition pour application topique comprenant dans un milieu physiologiquement acceptable, des fibres et au moins un copolymère comportant des groupements carboxylates et des groupements polydiméthylsiloxanes.

2. Composition selon la revendication 1, **caractérisée en ce que** les fibres ont une longueur (L) allant de 1 µm à 10 mm.

3. Composition selon la revendication 1 ou 2, **caractérisée en ce que** les fibres ont une section comprise dans un cercle de diamètre (D) allant de 1 nm à 100 µm.

4. Composition selon l'une quelconque des revendications précédentes, **caractérisée en ce que** les fibres ont un facteur de forme (L/D) allant de 5 à 150.

5. Composition selon l'une quelconque des revendications précédentes, **caractérisée en ce que** les fibres ont un titre allant de 0,15 à 30 deniers.

6. Composition selon l'une quelconque des revendications précédentes, **caractérisée en ce que** les fibres sont choisies parmi les fibres de soie, de coton, de liège, de canne à sucre, de laine, de lin, de cellulose, de polyamide, de cellulose modifiée, de poly-(p-phénylène téréphtalamide), en acrylique, de polyoléfine, de verre, de silice, d'aramide, de carbone, de Téflon® , de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges.

7. Composition selon l'une quelconque des revendications précédentes, **caractérisée en ce que** les fibres sont traitées et/ou enrobées.

8. Composition selon l'une quelconque des revendications précédentes, **caractérisée en ce que** les fibres sont des fibres d'origine synthétique.

9. Composition selon l'une quelconque des revendications précédentes, **caractérisée en ce que** les fibres sont choisies parmi les fibres de polyamide, les fibres de poly-(p-phénylènetéréphtalamide), les fibres de coton et leurs mélanges.

10. Composition selon l'une quelconque des revendications précédentes, **caractérisée en ce que** les fibres sont présentes en une quantité allant de 0,1 à 50 % en poids et de préférence de 0,5 à 30 % en poids par rapport au poids total de la composition.

11. Composition selon l'une quelconque des revendications précédentes, **caractérisée en ce que** le copolymère est obtenu à partir de (a) un ou plusieurs monomères carboxyliques, et (b) une ou plusieurs chaînes polydiméthylsiloxane comportant au moins un groupe radical polymérisable.

12. Composition selon la revendication précédente, **caractérisée en ce que** le monomère (a) est choisi parmi l'acide acrylique, l'acide méthacrylique, l'acide maléique, l'acide fumarique, l'acide itaconique, l'acide crotonique, leurs esters, et leurs mélanges.

13. Composition selon l'une quelconque des revendications précédentes, **caractérisée en ce que** les groupements carboxylates du copolymère sont choisis parmi l'acide acrylique, l'acide méthacrylique, les acrylates ou méthacrylates de méthyle, d'éthyle, de stéaryle, de butyle, d'éthyl-2-hexyle, et leurs mélanges.

14. Composition selon l'une quelconque des revendications précédentes, **caractérisée en ce que** le copolymère est obtenu par polymérisation radicalaire d'un polydiméthylsiloxane comportant au moins un groupe radical polymérisable et d'au moins un monomère carboxylique.

15. Composition selon l'une quelconque des revendications précédentes, **caractérisée en ce que** le copolymère a un poids moléculaire allant de 5000 à 200 000.

16. Composition selon l'une quelconque des revendications précédentes, **caractérisée en ce que** la quantité de copolymère va de 0,01 à 20 % en poids de matière active, par rapport au poids total de la composition.

17. Composition selon l'une quelconque des revendications précédentes, **caractérisée en ce qu'**elle comprend en outre une phase huileuse.

18. Composition selon la revendication précédente, **caractérisée en ce que** la phase huileuse contient au moins un corps gras choisi parmi les huiles liquides à température ambiante, volatiles ou non, les cires, les gommes et les corps gras pâteux, et leurs mélanges.

19. Composition selon l'une quelconque des revendications précédentes, **caractérisée en ce qu'**elle contient, en outre, une phase aqueuse.

20. Composition selon l'une quelconque des revendications précédentes, **caractérisée en ce qu'**elle contient, en outre, au moins une charge et/ou un agent épaississant lipophile.

21. Composition selon l'une quelconque des revendications précédentes, **caractérisée en ce qu'**elle contient en outre au moins une charge sphérique.

22. Composition selon l'une quelconque des revendications précédentes, **caractérisée en ce qu'**elle se présente sous forme d'un gel huileux ou aqueux ou d'émulsion huile-dans-eau ou eau-dans-huile ou multiple.

23. Composition selon l'une quelconque des revendications précédentes, **caractérisée en ce qu'**elle constitue une composition dermatologique ou cosmétique.

24. Composition selon l'une quelconque des revendications précédentes, **caractérisée en ce qu'**elle constitue une composition de traitement ou de soin de la peau, des fibres kératiniques, des ongles ou des lèvres, une composition de protection solaire ou de bronzage artificiel, un produit nettoyant ou démaquillant de la peau, des cheveux, des sourcils ou des cils, un produit déodorant, un produit parfumant, un fond de teint, un blush, un fard à joues ou à paupières, un mascara, un eye-liner, un produit anti-cernes en stick, un vernis à ongle, un rouge à lèvres ou un brillant à lèvres.

25. Utilisation cosmétique d'une composition cosmétique selon l'une quelconque des revendications 1 à 22, pour le soin, le traitement, le nettoyage et/ou le maquillage des matières kératiniques.

26. Procédé cosmétique de soin ou de traitement des matières kératiniques des êtres humains, comprenant l'application sur ces matières kératiniques d'une composition cosmétique selon l'une quelconque des revendications 1 à 22.

27. Utilisation d'un copolymère comportant des groupements carboxylates et des groupements polydiméthylsiloxanes, dans une composition pour application topique contenant des fibres, pour assurer une dispersion homogène des fibres dans la dite composition et pour stabiliser ladite composition.

28. Utilisation d'un copolymère comportant des groupements carboxylates et des groupements polydiméthylsiloxanes, dans une composition pour application topique contenant des fibres, comme agent dispersant.

29. Utilisation cosmétique d'un copolymère comportant des groupements carboxylates et des groupements polydiméthylsiloxanes, dans une composition cosmétique contenant des fibres, pour obtenir un dépôt homogène de la dite composition sur les matières kératiniques.

## Patentansprüche

1. Zusammensetzung zur topischen Anwendung, die in einem physiologisch akzeptablen Medium Fasern und mindestens ein Copolymer enthält, das Carboxylatgruppen und Polydimethylsiloxangruppen aufweist.

2. Zusammensetzung nach Anspruch 1, **dadurch gekennzeichnet, dass** die Fasern eine Länge (L) von 1 µm bis 10 mm besitzen.

3. Zusammensetzung nach Anspruch 1 oder 2, **dadurch gekennzeichnet, dass** die Fasern einen Querschnitt aufweisen, der in einen Kreis mit einem Durchmesser (D) von 1 nm bis 100 µm eingeschrieben werden kann.

4. Zusammensetzung nach einem der vorhergehenden Ansprüche, **dadurch gekennzeichnet, dass** die Fasern einen Formfaktor (L/D) von 5 bis 150 aufweisen.

5. Zusammensetzung nach einem der vorhergehenden Ansprüche, **dadurch gekennzeichnet, dass** die Fasern eine Feinheit von 0,15 bis 30 Denier besitzen.

6. Zusammensetzung nach einem der vorhergehenden Ansprüche, **dadurch gekennzeichnet, dass** die Fasern unter den Seidenfasern, Baumwollfasern, Korkfasern, Zuckerrohrfasern, Wollfasern, Leinenfasern, Cellulosefasern, Polyamidfasern, Fasern aus modifizierter Cellulose, Poly-(p-phenylenterephthalamid)-Fasern, Acrylfasern, Polyolefinfasern, Glasfasern, Siliciumdioxidfasern, Aramidfasern, Kohlenstofffasern, Teflon®-Fasern, Fasern aus unlöslichem Collagen, Polyesterfasern, Polyvinylchloridfasern, Polyvinylidenchloridfasem, Polyvinylalkoholfasem, Polyacrylnitrilfasem, Chitosanfasern, Polyurethanfasern, Polyethylenphthalatfasem, Fasern, die aus einem Gemisch von Polymeren bestehen, synthetischen resorbierbaren Fasern und deren Gemischen ausgewählt sind.

7. Zusammensetzung nach einem der vorhergehenden Ansprüche, **dadurch gekennzeichnet, dass** die Fasern behandelt und/oder umhüllt sind.

8. Zusammensetzung nach einem der vorhergehenden Ansprüche, **dadurch gekennzeichnet, dass** es sich bei den Fasern um Fasern synthetischer Herkunft handelt.

9. Zusammensetzung nach einem der vorhergehenden Ansprüche, **dadurch gekennzeichnet, dass** die Fasern unter den Polyamidfasern, Poly-(p-phenylenterephthalamid)-Fasern, Baumwollfasern und deren Gemischen ausgewählt sind.

10. Zusammensetzung nach einem der vorhergehenden Ansprüche, **dadurch gekennzeichnet, dass** die Fasern in einer Menge von 0,1 bis 50 Gew.-% und vorzugsweise 0,5 bis 30 Gew.-%, bezogen auf das Gesamtgewicht der Zusammensetzung, enthalten sind.

11. Zusammensetzung nach einem der vorhergehenden Ansprüche, **dadurch gekennzeichnet, dass** das Copolymer aus (a) einem oder mehreren Carboxymonomeren und (b) einer oder mehreren Polydimethylsiloxanketten, die mindestens eine polymerisierbare Gruppe enthalten, hergestellt wird.

12. Zusammensetzung nach dem vorhergehenden Anspruch, **dadurch gekennzeichnet, dass** das Monomer (a) unter Acrylsäure, Methacrylsäure, Maleinsäure, Fumarsäure, Itaconsäure, Crotonsäure und deren Estern und deren Gemischen ausgewählt ist.

13. Zusammensetzung nach einem der vorhergehenden Ansprüche, **dadurch gekennzeichnet, dass** die Carboxylatgruppen des Copolymers unter Acrylsäure, Methacrylsäure, Methylacrylat, Ethylacrylat, Stearylacrylat, Butylacrylat, 2-Ethylhexylacrylat, Methylmethacrylat, Ethylmethacrylat, Stearylmethacrylat, Butylmethacrylat, 2-Ethylhexylmethacrylat und deren Gemischen ausgewählt sind.

14. Zusammensetzung nach einem der vorhergehenden Ansprüche, **dadurch gekennzeichnet, dass** das Copolymer durch radikalische Polymerisation eines Polydimethylsiloxans, das mindestens eine radikalisch polymerisierbare Gruppe aufweist, und mindestens eines Carboxymonomers hergestellt wird.

15. Zusammensetzung nach einem der vorhergehenden Ansprüche, **dadurch gekennzeichnet, dass** das Copolymer ein Molekulargewicht von 5.000 bis 200.000 aufweist.

16. Zusammensetzung nach einem der vorhergehenden Ansprüche, **dadurch gekennzeichnet, dass** der Mengenanteil des Copolymers im Bereich von 0,01 bis 20 Gew.-% Wirkstoff, bezogen auf das Gesamtgewicht der Zusammensetzung, liegt.

17. Zusammensetzung nach einem der vorhergehenden Ansprüche, **dadurch gekennzeichnet, dass** sie ferner eine Ölphase aufweist.

18. Zusammensetzung nach dem vorhergehenden Anspruch, **dadurch gekennzeichnet, dass** die Ölphase mindestens eine Fettsubstanz enthält, die unter den bei Umgebungstemperatur flüssigen, flüchtigen oder nicht flüchtigen Ölen, Wachsen, Gummis und pastösen Fettsubstanzen und deren Gemischen ausgewählt ist.

19. Zusammensetzung nach einem der vorhergehenden Ansprüche, **dadurch gekennzeichnet, dass** sie ferner eine wässerige Phase aufweist.

20. Zusammensetzung nach einem der vorhergehenden Ansprüche, **dadurch gekennzeichnet, dass** sie ferner mindestens einen Füllstoff und/oder ein lipophiles Verdickungsmittel enthält.

21. Zusammensetzung nach einem der vorhergehenden Ansprüche, **dadurch gekennzeichnet, dass** sie ferner mindestens einen kugelförmigen Füllstoff enthält.

22. Zusammensetzung nach einem der vorhergehenden Ansprüche, **dadurch gekennzeichnet, dass** sie als öliges Gel, wässeriges Gel, Öl-in-Wasser-Emulsion, Wasser-in-Öl-Emulsion oder multiple Emulsion vorliegt.

23. Zusammensetzung nach einem der vorhergehenden Ansprüche, **dadurch gekennzeichnet, dass** es sich um eine dermatologische oder kosmetische Zusammensetzung handelt.

24. Zusammensetzung nach einem der vorhergehenden Ansprüche, **dadurch gekennzeichnet, dass** es sich um Zusammensetzungen zur Behandlung oder Pflege der Haut, der Keratinfasern, der Nägel oder der Lippen, Zusammensetzungen zum Sonnenschutz oder zur künstlichen Bräunung, Produkte zur Reinigung oder zum Abschminken der Haut, der Haare, der Wimpern oder der Augenbrauen, desodorisierende Produkte, parfümierende Produkte, Make-up, Blush, Wangenrouge, Lidschatten, Mascara, Eyeliner, Produkte gegen Augenringe in Stiftform, Nagellacke, Lippenstifte oder Lippenglanz handelt.

25. Kosmetische Verwendung einer kosmetischen Zusammensetzung nach einem der Ansprüche 1 bis 22 zur Pflege, zur Behandlung, zur Reinigung und/oder zum Schminken von Keratinsubstanzen.

26. Kosmetische Verfahren zur Pflege oder zur Behandlung von menschlichen Keratinsubstanzen, das umfasst, auf die Keratinsubstanzen eine kosmetische Zusammensetzung nach einem der Ansprüche 1 bis 22 aufzutragen.

27. Verwendung eines Copolymers, das Carboxylatgruppen und Polydimethylsiloxangruppen enthält, in einer Zusammensetzung zur topischen Anwendung, die Fasern enthält, um eine homogene Dispersion der Fasern in der Zusammensetzung zu gewährleisten und die Zusammensetzung zu stabilisieren.

28. Verwendung eines Copolymers, das Carboxylatgruppen und Polydimethylsiloxangruppen aufweist, in einer Zusammensetzung zur topischen Anwendung, die Fasern enthält, als Dispergiermittel.

29. Kosmetische Verwendung eines Copolymers, das Carboxylatgruppen und Polydimethylsiloxangruppen enthält, in einer kosmetischen Zusammensetzung, die Fasern enthält, um auf den Keratinsubstanzen ein homogenes Depot der Zusammensetzung zu bilden.

## Claims

1. Composition for topical application, comprising, in a physiologically acceptable medium, fibres and at least one copolymer comprising carboxylate groups and polydimethylsiloxane groups.

2. Composition according to Claim 1, **characterized in that** the fibres have a length (L) ranging from 1 µm to 10 mm.

3. Composition according to Claim 1 or 2, **characterized in that** the fibres have a cross section which is within a circle of diameter (D) ranging from 1 nm to 100 µm.

4. Composition according to any one of the preceding claims, **characterized in that** the fibres have a shape factor (L/D) ranging from 5 to 150.

5. Composition according to any one of the preceding claims, **characterized in that** the fibres have a yarn count ranging from 0.15 to 30 denier.

6. Composition according to any one of the preceding claims, **characterized in that** the fibres are chosen from silk fibre, cotton fibre, cork fibre, sugar cane fibre, wool fibre, flax fibre, cellulose fibre, polyamide fibre, modified cellulose fibre, poly(p-phenyleneterephthalamide) fibre, acrylic fibre, polyolefin fibre, glass fibre, silica fibre, aramide fibre, carbon fibre, Teflon® fibre, insoluble collagen fibre, polyester fibre, polyvinyl chloride fibre or polyvinylidene chloride fibre, polyvinyl alcohol fibre, polyacrylonitrile fibre, chitosan fibre, polyurethane fibre, polyethylene phthalate fibre, and fibres formed from a mixture of polymers, and resorbable synthetic fibres, and mixtures thereof.

7. Composition according to any one of the preceding claims, **characterized in that** the fibres are treated and/or coated.

8. Composition according to any one of the preceding claims, **characterized in that** the fibres are fibres of synthetic origin.

9. Composition according to any one of the preceding claims, **characterized in that** the fibres are chosen from polyamide fibres, poly(p-phenylene-terephthalamide) fibres and cotton fibres, and mixtures thereof.

10. Composition according to any one of the preceding claims, **characterized in that** the fibres are present in an amount ranging from 0.1% to 50% by weight and preferably from 0.5% to 30% by weight relative to the total weight of the composition.

11. Composition according to any one of the preceding claims, **characterized in that** the copolymer is obtained from (a) one or more carboxylic monomers, and (b) one or more polydimethylsiloxane chains comprising at least one polymerizable radical group.

12. Composition according to the preceding claim, **characterized in that** the monomer (a) is chosen from acrylic acid, methacrylic acid, maleic acid, fumaric acid, itaconic acid and crotonic acid, esters thereof, and mixtures thereof.

13. Composition according to any one of the preceding claims, **characterized in that** the carboxylate groups of the copolymer are chosen from acrylic acid, methacrylic acid and methyl, ethyl, stearyl, butyl or 2-ethylhexyl acrylates or methacrylates, and mixtures thereof.

14. Composition according to any one of the preceding claims, **characterized in that** the copolymer is obtained by free-radical polymerization of a polydimethylsiloxane comprising at least one polymerizable radical group and of at least one carboxylic monomer.

15. Composition according to any one of the preceding claims, **characterized in that** the copolymer has a molecular weight ranging from 5000 to 200 000.

16. Composition according to any one of the preceding claims, **characterized in that** the amount of copolymer ranges from 0.01% to 20% by weight of active material relative to the total weight of the composition.

17. Composition according to any one of the preceding claims, **characterized in that** it also comprises an oily phase.

18. Composition according to the preceding claim, **characterized in that** the oily phase contains at least one fatty substance chosen from volatile or non-volatile oils that are liquid at room temperature, waxes, gums and pasty fatty substances, and mixtures thereof.

19. Composition according to any one of the preceding claims, **characterized in that** it also contains an aqueous phase.

20. Composition according to any one of the preceding claims, **characterized in that** it also contains at least one filler and/or one lipophilic thickener.

21. Composition according to any one of the preceding claims, **characterized in that** it also contains at least one spherical filler.

22. Composition according to any one of the preceding claims, **characterized in that** it is in the form of an oily or aqueous gel or of an oil-in-water or water-in-oil or multiple emulsion.

23. Composition according to any one of the preceding claims, **characterized in that** it is a dermatological or cosmetic composition.

24. Composition according to any one of the preceding claims, **characterized in that** it constitutes a treatment or care composition for the skin, keratin fibres, the nails or the lips, an antisun or artificial tanning composition, a cleansing or make-up-removing product for the skin, the hair, the eyebrows or the eyelashes, a deodorant product, a fragrancing product, a foundation, a blusher, a face powder, an eyeshadow, a mascara, an eyeliner, a concealer stick, a nail varnish, a lipstick or a lip gloss.

25. Cosmetic use of a cosmetic composition according to any one of Claims 1 to 22, for caring for, treating, cleansing and/or making up keratin materials.

26. Cosmetic process for caring for or treating keratin materials of human beings, comprising the application to these keratin materials of the cosmetic composition according to any one of Claims 1 to 22.

27. Use of a copolymer comprising carboxylate groups and polydimethylsiloxane groups, in a composition for topical application containing fibres, to ensure a homogeneous dispersion of the fibres in the said composition and to stabilize the said composition.

28. Use of a copolymer comprising carboxylate groups and polydimethylsiloxane groups, in a composition for topical application containing fibres, as a dispersant.

29. Cosmetic use of a copolymer comprising carboxylate groups and polydimethylsiloxane groups, in a cosmetic composition containing fibres, to obtain a homogeneous deposit of the said composition on keratin materials.
